# EUROPEAN PATENT APPLICATION

(11) **EP 1 939 302 A1**
(43) Date of publication of application: **02.07.2008**
(21) Application number: 06822019.3
(22) Date of filing: 16.10.2006
(51) Int. Cl.: C12P 19/00, A01G 33/00

(54) **PROCESS FOR PRODUCING -CHITIN**

(30) Priority: 17.10.2005 JP 2005301981
(71) Applicant: Yamaha Hatsudoki Kabushiki Kaisha, Iwata-shi, Shizuoka 438-8501 (JP)
(72) Inventor: TSUJI, Shinji, Iwata-shi Shizuoka 438-8501 (JP); YAMAUCHI, Ichiro, Iwata-shi Shizuoka 438-8501 (JP); KONDOU, Yutaka, Iwata-shi Shizuoka 438-8501 (JP)
(74) Representative: Schlich, George William
(86) International application number: PCT/JP2006/320993
(87) International publication number: WO 2007/046517

(57) **Abstract**

The method of the present invention for producing β-chitin comprises a step of cultivating an alga capable of producing β-chitin using a medium that contains nitrogen (N), phosphorus (P), silicon (Si), and selenium (Se), wherein the weight ratio of phosphorus to nitrogen (P/N) is 0.1 or above, the weight ratio of silicon to nitrogen (Si/N) is 0.1 or above, and nitrogen (N) is contained at 160 ppm or more. It is preferable that selenium (Se) is present in the medium at a concentration of 0.08 ppm to 0.008 ppb.

## Description

### Technical Field

The present invention relates to a method for producing β-chitin. More specifically, the present invention provides a method for efficiently producing β-chitin from an alga, and in particular, an alga belonging to the genus *Thalassiosira.*

### Background Art

Chitin is a naturally occurring polysaccharide made of N-acetyl-β-D-glucosamine units linked together in a 1,4 mode, and chitosan is the deacetylated form of chitin. Chitin and chitosan are extracted from the shells of crab and shrimp, for example, and also are produced from algae (for example, WO 95/15343 (Japanese Laid-Open Patent Publication (Tokuhyo) No. 9-506126)).

Chitin and chitosan have various properties, including antibacterial properties, moisture retention properties, thickening properties, and biocompatibility, and thus are used in fields such as the fields of biomedical materials, pharmaceuticals, cosmetics, fibers, food products, and agriculture (Fiscal year 2003 Tokkyo Ryutsu Shien Chart (Chemical 19) Chitin and Chitosan Riyo Gijutsu, March 2004, National Center for Industrial Property Information and Training).

Chitin forms a unique crystalline structure due to hydrogen bonding between acetamide groups or between acetamide groups and hydroxyl groups of the N-acetyl-β-D-glucosamine residues that constitute the chitin molecule. Chitin includes α-chitin, in which the chitin molecules are ordered alternately, and β-chitin, in which the chitin molecules are parallel to one another and are oriented in the same direction. It should be noted that α-chitin and β-chitin may also be written as alpha-chitin and beta-chitin, respectively. The fact that α-chitin and alpha-chitin are identical and that β-chitin and beta-chitin are identical should be sufficiently apparent to those skilled in the art.

Naturally occurring chitin, which is typically produced from the shells described above, has an α-crystal structure that is hard and most stable.

On the other hand, β-chitin has a comparatively loose structure, in which sheet-like chitin molecular chains are layered, and it has been reported that β-chitin can form inclusion compounds (Chitin and Chitosan Research, Volume 8, Number 2, p.186, 2002, and Chitin and Chitosan Research, Volume 9, Number 2, p.100, 2003). Such inclusion compounds can contribute to novel applications for chitin, such as the construction of novel drug delivery systems. It is known that β-chitin may be prepared from squid gladius, or may be prepared from diatoms by the method described in WO 95/15343. However, the amount of β-chitin produced from diatoms is low, and there is a limit to the amount that can be supplied.

In general, the cultivation of algae is often carried out by supplying nitrogen and phosphorous compounds to seawater or artificial seawater under irradiation with natural light or artificial light. For example, Japanese Laid-Open Patent Publication No. 2004-187675 describes a medium that enables the diatoms belonging to the genus *Chaetoceraceae* and the genus *Phaeodactylum* to be cultivated at high concentration. The publication also describes that diatoms can be practically used as feed for marine seeds by using the medium. However, this publication focuses only on the efficiency of growth of the algae, and there is no description of the efficiency of β-chitin production by the cultivation of algae, that is to say, of the media suitable for the production of β-chitin.

### Disclosure of Invention

It is an object of the invention to provide a method for efficiently producing β-chitin by the cultivation of algae.

The present invention provides a method for producing β-chitin, comprising: cultivating an alga capable of producing β-chitin, using a medium that contains nitrogen (N), phosphorus (P), silicon (Si), and selenium (Se); wherein the weight ratio of phosphorus to nitrogen (P/N) is 0.1 or above, the weight ratio of silicon to nitrogen (Si/N) is 0.1 or above, and nitrogen (N) is present at a concentration of 160 ppm or more.

In one embodiment, the alga is an alga belonging to the genus *Thalassiosira.*

In a separate embodiment, the concentration of selenium (Se) in the medium is from 0.08 ppm to 0.008 ppb.

In a yet further separate embodiment, the concentration of phosphorus (P) in the medium is 20 ppm or more, and the concentration of silicon (Si) is 60 ppm or more.

According to the present invention, the concentration of β-chitin in the cultivated algae is 5 or more times greater than the concentration obtained according to conventional methods, and thus it is possible to produce β-chitin more efficiently.

### Brief Description of the Drawings

Fig. 1 is a graph showing the change over time in the number of cells in the culture suspension in a case where *Thalassiosira* are cultivated at 30°C in Example 1, Comparative Example 1, and Comparative Example 2.
Fig. 2 is a graph showing the change over time in the β-chitin content in the culture suspension in a case where *Thalassiosira* are cultivated at 30°C in Example 1, Comparative Example 1, and Comparative Example 2.
Fig. 3 is a graph showing the change over time in the number of cells in the culture suspension in a case where *Thalassiosira* are cultivated at 25°C in Example 2 and Comparative Example 3.
Fig. 4 is a graph showing the change over time in the β-chitin content in the culture suspension in a case where *Thalassiosira* are cultivated at 25°C in Example 2 and Comparative Example 3.
Fig. 5 is a graph showing the change over time in the β-chitin content in the culture suspension in a case where *Thalassiosira* are cultivated at 30°C in Examples 3 to 7 and Comparative Example 4.

### Best Mode for Carrying Out the Invention

In the present specification, chitin is used to refer not only to poly-β-1,4-N-acetylglucosamine, which is a polymer of N-acetyl-β-D-glucosamine, but also to poly-β-1,4-N-acetylglucosamine derivatives in which a portion of the acetyl groups have been eliminated.

### Algae Capable of Producing β-Chitin

Examples of algae capable of producing β-chitin that are used in the method of the present invention include algae belonging to the genus *Thalassiosira,* the genus *Coscinodiscus,* and the genus *Cyclotella,* but there is no limitation thereto.

Of these, algae of the genus *Thalassiosira* are preferably used. Examples of algae belonging to the genus *Thalassiosira* include *Thalassiosira nitzschoides* (*T. nitzschoides*), *Thalassiosira aestivalis* (*T. aestivalis*), *Thalassiosira antarctica* (*T. antarctica*), *Thalassiosira deciphens* (*T. deciphens*), *Thalassiosira eccentrica* (*T. eccentrica*), *Thalassiosira floridana* (*T. floridana*), *Thalassiosira fluviatilis* (*T. fluviatilis*), *Thalassiosira gravida* (*T. gravida*), *Thalassiosira guillardii* (*T. guillardii*), *Thalassiosira hyalina* (*T. hyalina*), *Thalassiosira minima* (*T. minima*), *Thalassiosira nordenskioldii* (*T. nordenskioldii*), *Thalassiosira oceanica* (*T. oceanica*), *Thalassiosira polychorda* (*T. polychorda*), *Thalassiosira pseudonana* (*T. pseudonana*), *Thalassiosira rotula* (*T. rotula*), *Thalassiosira tubifera* (*T. tubifera*), *Thalassiosira tumida* (*T. tumida*), and *Thalassiosira weissflogii* (*T. weissflogii*).

These algae can be obtained from The Provasoli-Guillard National Center for Culture of Marine Phytoplankton (CCMP).

### Medium for Producing β-Chitin

The medium used in the method of the present invention contains nitrogen (N), phosphorus (P), silicon (Si), and Selenium (Se), the weight ratio of phosphorus to nitrogen (P/N) in the medium is 0.1 or above, the weight ratio of silicon and nitrogen is (Si/N) is 0.1 or above, and nitrogen (N) is present at a concentration of 160 ppm or more.

For the purpose of algae growth and production of β-chitin, the medium used in the present invention contains nitrogen (N) at a concentration of 160 ppm or more, as mentioned above. Nitrogen (N) is preferably contained at a concentration of 200 ppm or more, and more preferably 300 ppm or more. Phosphorus (P) is preferably contained at a concentration of 20 ppm or more, more preferably 30 ppm or more, and even more preferably 40 ppm or more. Silicon (Si) is preferably contained at a concentration of 60 ppm or more, more preferably 80 ppm or more, and even more preferably 100 ppm or more.

As the compounds containing nitrogen (N), phosphorus (P), and silicon (Si), compounds that are generally used in the cultivation of algae may be used. As nitrogen-containing compounds, ammonium salts (such as ammonium nitrate and ammonium sulfate) and nitrates (such as sodium nitrate and potassium nitrate) may be used. Examples of phosphorus-containing compounds include sodium hydrogen phosphate and potassium hydrogen phosphate. As a compound containing both nitrogen and phosphate, ammonium phosphate may be used, for example. Examples of silicon-containing compounds include sodium silicate and potassium silicate. The compounds listed above are illustrative examples, and there is no limitation thereto.

The medium that is used in the method of the present invention contains selenium (Se). In order to efficiently grow algae and produce β-chitin, the selenium content in a selenium-containing medium is preferably from 0.08 ppm to 0.008 ppb, and more preferably from 0.08 ppm to 0.08 ppb. The selenium concentration in an algal culture suspension obtained by using a selenium-containing medium is preferably 0.075 ppm to 0.0075 ppb, and more preferably 0.075 ppm to 0.075 ppb.

The medium used in the method of the present invention may optionally include trace metals (such as Fe, Cu, Zn, Co, Mn, Mo, Ca, and Mg) or vitamins (such as vitamin B12 and biotin) commonly used in the cultivation of algae.

The pH of the medium that is used in the present invention is preferably in the range of 6 to 9.

### Cultivation and Production of β-Chitin

β-chitin is produced by cultivating in the above medium the algae capable of producing β-chitin, Cultivation is performed under irradiation with light while bubbling carbon dioxide. There is no particular limitation on the culture temperature as long as it is a temperature at which the algae can grow, although generally the temperature is 15°C to 35°C. In the case of algae of the genus Thalassiosira, the algae are cultivated at a temperature from 25 to 32°C, and preferably around 30°C. There is no particular limitation on the culture time.

After cultivation has ended, β-chitin is collected, for example, by the following procedure, which includes: a step of mechanically stirring the culture suspension to cleave the β-chitin spines from the algal cells; a step of precipitating the algal cells by separation means such as centrifugation to remove the algal cells; a step of dispersing the thus obtained algal cell-free solution in an alkaline solution (such as a potassium hydroxide solution); and a step of collecting a solid material from the dispersion solution, by using a membrane filter, for example. The crude β-chitin collected is further purified by purification means commonly used by those skilled in the art.

### EXAMPLES

Hereinafter, the present invention will be described by means of examples. However, it goes without saying that the present invention is not limited to these examples.

Table 1 shows the compositions of the media used in the following examples and comparative examples.

**Table 1**

| | Medium A | Medium B | Medium C | Medium D | Medium E | Medium F |
|---|---|---|---|---|---|---|
| NaNO₃ | 1.4g/L | 1.4g/L | 1.4g/L | 1.4g/L | 1.4g/L | 1.4g/L |
| (NH₄)₂SO₄ | 0.48 g/ L | 0.48 g/ L | 0.48 g/ L | 0.48 g/ L | 0.48 g/ L | 0.48 g/ L |
| KH₂PO₄ | 0.198 g/L | 0.198 g/L | 0.198 g/L | 0.198 g/L | 0.198 g/L | 0.198 g/L |
| Vitamine B12 | 0.06 mg/L | 0.06 mg/L | 0.06 mg/L | 0.06 mg/L | 0.06 mg/L | 0.06 mg/L |
| Biotin | 0.03 mg/L | 0.03 mg/L | 0.03 mg/L | 0.03 mg/L | 0.03 mg/L | 0.03mg/L |
| Thiamine hydrochloride | 6mg/ L | 6mg/ L | 6mg/ L | 6mg/ L | 6mg/ L | 6mg/ L |
| CLEWAT32 | 0.1 g/ L | 0.1 g/ L | 0.1 g/ L | 0.1 g/ L | 0.1 g/ L | 0.1 g/ L |
| Fe-EDTA | 0.066 g/ L | 0.066 g/ L | 0.066 g/ L | 0.066 g/ L | 0.066 g/ L | 0.066 g/ L |
| Na₂SiO₃·9H₂O | 1.3g/L | 1.3g/L | 1.3g/L | 1.3g/L | 1.3g/L | 1.3g/L |
| SEALIFE | 35.0g/L | 35.0g/L | 35.0g/L | 35.0g/L | 35.0g/L | 35.0g/L |
| NaHCO₃ | 1.0g/L | 1.0g/L | 1.0g/L | 1.0g/L | 1.0g/L | 1.0g/L |
| H₂SeO₃ | 0.0013 m g/ L | - | 0.13 m g/ L | 0.013 m g/ | L 0.00013 m g/ | L 0.000013 m g/ L |

The various media differed only in the concentrations of selenium, and the other components and their concentrations were identical.

The amount of algal cell and the amount of β-chitin were measured as follows.

### Measuring the Algal Cell Amount

The amount of algal cells in the culture suspension was measured as follows using an Erma hemocytometer (made by ERMA INC). An undiluted culture suspension was poured into the calculation chamber of the hemocytometer by a Pasteur pipette at the start of cultivation. Until 48 hours after the start of cultivation, the undiluted culture suspension was used to measure the number of cells in the same manner as above. The number of cells in the calculation chamber was measured by microscope (magnification: 10×10). From the 72nd hour after the start of cultivation, the culture suspension was diluted by a factor of 10 using artificial seawater containing salts at a quarter of concentration of seawater, and the amount of algal cells was measured by the same method.

### Measuring the β-chitin Amount

The amount of β-chitin in the algal cells was measured as follows. First, 5 mL of the culture suspension was mechanically stirred to cleave the β-chitin spines from the algal cells. After stirring, the culture suspension was centrifuged at 1,500 x g for 20 minutes. Since the culture suspension was viscous and the algal cells could not be completely separated, the supernatant was obtained by decantation and was subjected to further centrifugation at 2,000 x g for 20 minutes, and the supernatant containing the β-chitin spines was collected. The supernatant was centrifuged at 11,000 x g for 30 minutes to give the precipitate. The precipitate included β-chitin spines and other components, for example proteins and ash components. To the obtained precipitate 5 wt% potassium hydroxide solution was added to disperse the precipitate, and the thus obtained dispersion was allowed to stand at room temperature for 12 hours to dissolve the proteins, which were contaminants. The β-chitin spines were dispersed in the potassium hydroxide solution without dissolving. Then, the dispersed β-chitin was filtered by a polytetrafluoroethylene (PTFE) membrane filter with a 1.0 µm pore size to collect β-chitin. The collected β-chitin was again dispersed in methanol and maintained at 60°C for two hours. The β-chitin was then again filtered by a PTFE membrane filter. The filtrate was dispersed in a 0.1 mol/L aqueous hydrochloric acid and boiled for one hour to dissolve the ash component, and then the β-chitin was collected by filtration. The collected β-chitin was dispersed in a 0.34 wt% aqueous sodium chlorite (NaClO₂) and maintained at 80°C for two hours. The dispersion was filtered to collect the insoluble matter, which was then dispersed in a 1 wt% aqueous hydrofluoric acid, and allowed to stand at room temperature for 12 hours. Then, the insoluble matter was again collected by filtration, and dried at room temperature to give a hydrate of purified β-chitin. The β-chitin hydrate was dried at 105°C for two hours to obtain anhydrous β-chitin and the weight of the anhydrate obtained was measured, and taken as the amount of β-chitin.

### Example 1

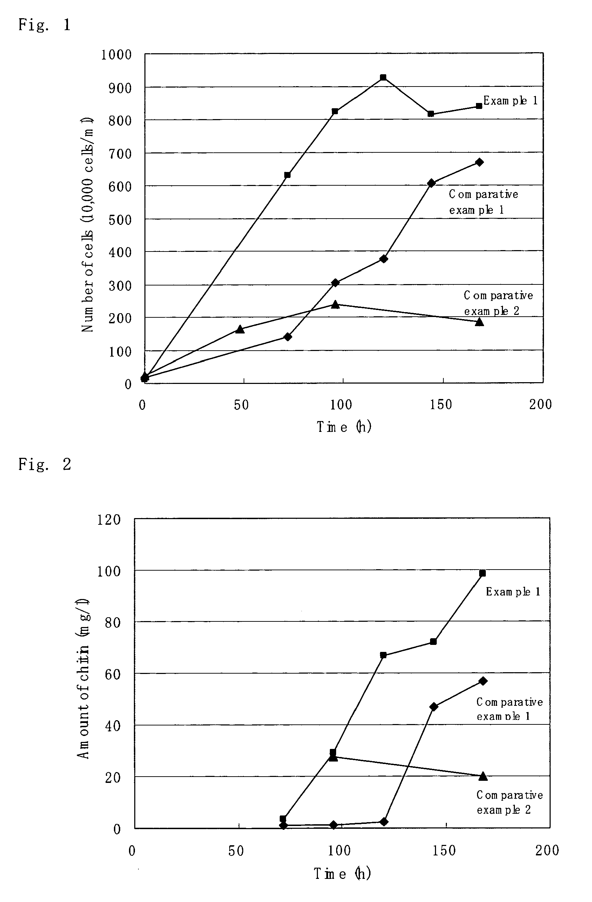

First, 1 L of the medium A listed in Table 1 was placed into a 1.5 L glass flat culture flask, and 1.0 g sodium bicarbonate (NaHCO₃) was added thereto to prepare a culture solution A. Then, 50 mL culture of the *Thalassiosira weissflogii* CCMP 1051 strain, which was cultivated in advance on the medium B that does not contain selenium (Se), was inoculated into the culture solution A. The cultivation was performed at 30°C for 168 hours while stirring under irradiation with light at a photon density of approximately 200 µmol/m²/sec using a fluorescent lamp while bubbling air containing 3 vol% carbon dioxide into the culture suspension. The changes over time in the number of cells and the β-chitin content in the culture suspension are shown in Figs. 1 and 2, respectively. It should be noted that the selenium concentration in the culture suspension of Example 1 was 0.00075 ppm.

### Comparative Example 1

The *Thalassiosira weissflogii* CCMP 1051 strain was cultivated in the same manner as in Example 1, except that the medium B (which does not contain selenium) was used instead of the medium A. The changes over time in the number of cells and the β-chitin content in the culture suspension are shown in Figs. 1 and 2, respectively.

### Comparative Example 2

The *Thalassiosira weissflogii* CCMP 1051 strain was cultivated in the same manner as in Example 1, except that the medium described in WO 95/15343 was used. It should be noted that the medium described in WO 95/15343 is prepared based on seawater, and contains NaNO₃ at 75 mg/L, NaH₂PO₄·H₂O at 5 mg/L, Na₂SiO₃·9H₂O at 30 mg/L, and selenium (Se) compounds at 0.0013 mg/L, and also contains the same trace elements as those described in Example 1. The changes over time in the number of cells and the β-chitin content in the culture suspension are shown in Figs. 1 and 2, respectively. It should be noted that the selenium concentration in the culture suspension of Comparative Example 2 was 0.00075 ppm.

By comparing Example 1 and Comparative Example 2 (in which the medium had the same components as that of WO 95/15343), it is found that although both media contained selenium (Se), the medium used in Example 1, which contained components other than selenium at specific proportions, promoted the growth of *Thalassiosira* and increased the efficiency of β-chitin production. From the results of Example 1 and Comparative Example 1, it is found that the presence of selenium (Se) in media otherwise having the same compositions promoted the growth of *Thalassiosira* and increased the efficiency of β-chitin production.

From these observations, it is concluded that by addition of selenium (Se) to a medium containing nitrogen (N), phosphorus (P), and silicon (Si) in specific proportions, the effect of promoting the growth of *Thalassiosira* and increasing the efficiency of β-chitin production can be attained.

### Example 2

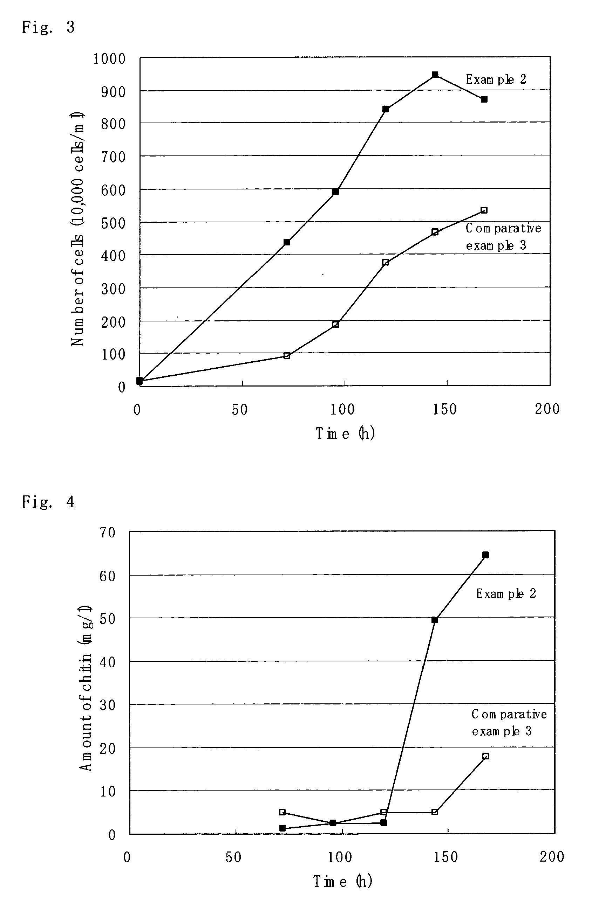

Cultivation was performed in the same manner as in Example 1, except that the culture temperature was 25°C. The changes over time in the number of cells and the β-chitin content in the culture suspension are shown in Figs. 3 and 4, respectively.

### Comparative Example 3

Cultivation was performed in the same manner as in Example 1, except that medium B was used instead of medium A and the culture temperature was 25°C. The changes over time in the number of cells and the β-chitin content in the culture suspension are shown in Figs. 3 and 4, respectively.

Figs. 3 and 4 show that the presence of selenium (Se) in the medium promoted the growth of *Thalassiosira* and increased the efficiency of β-chitin production.

### Example 3

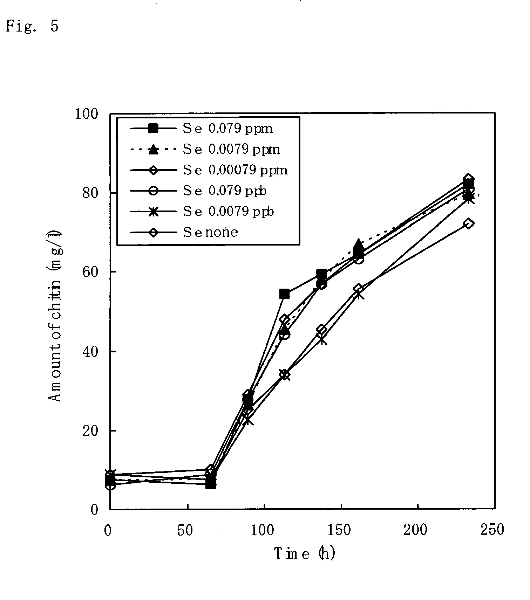

Cultivation was performed for 233 hours in the same manner as in Example 1, except that medium C was used instead of medium A. The change over time in the β-chitin content in the culture suspension is shown in Fig. 5. It should be noted that the selenium concentration in the culture suspension of Example 3 was 0.075 ppm.

### Example 4

Cultivation was performed for 233 hours in the same manner as in Example 1, except that medium D was used instead of medium A. The change over time in the β-chitin content in the culture suspension is shown in Fig. 5. It should be noted that the selenium concentration in the culture suspension of Example 4 was 0.0075 ppm.

### Example 5

Cultivation was performed for 233 hours in the same manner as in Example 1. The change over time in the β-chitin content in the culture suspension is shown in Fig. 5. It should be noted that the selenium concentration in the culture suspension of Example 5 was 0.00075 ppm.

### Example 6

Cultivation was performed for 233 hours in the same manner as in Example 1, except that medium E was used instead of medium A. The change over time in the β-chitin content in the culture suspension is shown in Fig. 5. It should be noted that the selenium concentration in the culture suspension of Example 6 was 0.075 ppb.

### Example 7

Cultivation was performed for 233 hours in the same manner as in Example 1, except that medium F was used instead of medium A. The change over time in the β-chitin content in the culture suspension is shown in Fig. 5. It should be noted that the selenium concentration in the culture suspension of Example 7 was 0.0075 ppb.

### Comparative Example 4

Cultivation was performed for 233 hours in the same manner as in Example 1, except that medium B (which did not contain Se) was used instead of medium A. The change over time in the β-chitin content in the culture suspension is shown in Fig. 5.

As shown in Fig. 5, cultivation for 233 hours results in greater production of β-chitin by the *Thalassiosira* cultivated in selenium-containing media. When cultivation was performed using media that contained selenium (Se) at a concentration of 0.079 ppm to 0.0079 ppb, that is, when the selenium concentration in the culture suspension was 0.075 ppm to 0.0075 ppb, β-chitin was produced in greater quantity by *Thalassiosira* than when the medium without selenium was used, regardless of the duration of culture time. The number of cells also tended to increase as the selenium concentration was higher (data are not shown).

### Industrial Applicability

According to the present invention, it is possible to produce β-chitin more efficiently. Since β-chitin can be used to construct novel drug delivery systems in fields such as the fields of biomedical materials, pharmaceuticals, cosmetics, fibers, food products, and agriculture, for example, it can contribute to the development of these fields.

## Claims

1. A method for producing β-chitin, comprising:
cultivating an alga capable of producing β-chitin in a medium that contains nitrogen (N), phosphorus (P), silicon (Si), and selenium (Se), wherein the weight ratio of phosphorus to nitrogen (P/N) is 0.1 or above, the weight ratio of silicon to nitrogen (Si/N) is 0.1 or above, and nitrogen (N) is present at a concentration of 160 ppm or more.

2. The method of claim 1, wherein the alga is an alga belonging to the genus *Thalassiosira.*

3. The method of claim 1, wherein the concentration of selenium (Se) in the medium is from 0.08 ppm to 0.008 ppb.

4. The method of any one of claims 1 to 3, wherein the concentration of phosphorus (P) in the medium is 20 ppm or more, and the concentration of silicon (Si) is 60 ppm or more.
